# EUROPÄISCHE PATENTSCHRIFT

(11) **EP 3 151 740 B1**
(45) Veröffentlichungstag und Bekanntmachung des Hinweises auf die Patenterteilung: **20.11.2024**
(21) Anmeldenummer: 15727558.7
(22) Anmeldetag: 01.06.2015
(51) Int. Cl.: A61B 5/026, A61B 5/00, A61B 7/04, A61B 5/01, A61M 1/36, A61B 5/1455

(54) **VORRICHTUNG ZUR NICHT INVASIVEN MESSUNG DES BLUTFLUSSES**
DEVICE FOR THE NON-INVASIVE MEASUREMENT OF BLOOD FLOW
DISPOSITIF DE MESURE NON INVASIVE DU FLUX SANGUIN

(30) Priorität: 06.06.2014 DE 102014008446
(43) Veröffentlichungstag der Anmeldung: 12.04.2017
(73) Patentinhaber: Fresenius Medical Care Deutschland GmbH, 61352 Bad Homburg (DE)
(72) Erfinder: KOPPERSCHMIDT, Pascal, 97456 Dittelbrunn (DE); MÜLLER, Carsten, 97502 Euerbach (DE)
(74) Vertreter: Herrmann, Uwe
(86) Internationale Anmeldenummer: PCT/EP2015/001107
(87) Internationale Veröffentlichungsnummer: WO 2015/185202

(56) Entgegenhaltungen:
- WO-A1-2012/163738
- WO-A2-2010/129528
- US-A1- 2002 099 286
- US-A1- 2012 323 127
- US-A1- 2013 041 235
- US-B1- 6 533 729
- US-B2- 7 368 855

## Beschreibung

Die vorliegende Erfindung betrifft eine Vorrichtung zur nicht invasiven Messung des Blutflusses durch einen Shunt eines Patienten.

Im Bereich der Blutreinigungsverfahren durch Hämodialyse, Hämofiltration oder Hämodiafiltration ist es bekannt, einen sogenannten Shunt einzusetzen, aus dem Blut zur extrakorporalen Reinigung abgezogen und in den Blut nach der extrakorporalen Reinigung zurückgeführt wird. Der Shunt weist im funktionsfähigen Zustand Durchflussraten des Blutes auf, die für die Durchführung der extrakorporalen Blutreinigung ausreichend sind. Die Funktion des Shunts ist somit für eine adäquate Hämodialyse etc. unabdingbar.

Während der Dialysetherapie hat ein nicht mehr ausreichend mit Blut versorgter Shunt Auswirkungen auf den Therapieverlauf, indem beispielsweise arterielle Druckalarme auftreten oder nur ein geringer erzielbarer alarmfreier Blutfluss erzielbar ist, und auch Auswirkungen auf den Therapieerfolg dahingehend, dass die Blutreinigung bzw. die Clearance eingeschränkt ist.

Ein Shunt kann durch einen chirurgischen Eingriff geschaffen werden, in dem eine Verbindung zwischen einer Arterie und einer Vene hergestellt wird, der als native Fistel bezeichnet wird. Bei einer anderen Shuntform kann mittels eines chirurgischen Eingriffs ein Zwischenstück eingesetzt werden, welches entweder aus einem röhrenförmigen, künstlichen Material oder aus einem Teil eines körpereigenen Blutgefäßes besteht und als Verbindung zwischen einer Arterie und einer Vene genutzt wird. Dieses Zwischenstück wird auch als Graft bezeichnet.

Da der Shunt durch die häufigen Punktionen über die Jahre stark beansprucht wird, ist eine Veränderung der Gefäßwand des Shunts nicht vermeidbar. So können Stenosen, Intimahyperplasien oder Thrombosen die Shuntfunktion beeinträchtigen oder diesen komplett unbrauchbar machen. Dies macht üblicherweise einen Klinikaufenthalt erforderlich, damit ein bestehender Shunt revidiert oder ein neuer Shunt angelegt werden kann.

Verändert ein Shunt sein Flussverhalten, so wird dies häufig erst bei Beginn der nächsten Dialysebehandlung bemerkt, beispielsweise durch wiederholt auftretende Druckalarme beim Einstellen der für den Patienten gewohnten Blutflussparameter oder auch später im Verlauf durch stark erniedrigte Clearance-Messwerte, die auf den geringen Durchfluss des Shunts und damit auf eine vergleichsweise geringe Menge des gereinigten Blutes zurückgeführt werden können. Unter Umständen kann die Dialysebehandlung aufgrund eines versiegelten Shunts nicht wie geplant durchgeführt werden und es muss unmittelbar und operativ ein alternativer Zugang, wie beispielsweise ein Shaldon-Katheter, ZVK etc. gelegt werden.

In der Phase zwischen den Behandlungen, d.h. in der interdialytischen Zeitspanne bleiben Funktionseinschränkungen des Shunts für den Patienten häufig unbemerkt.

Aus der US 6,725,072 B2 ist ein optischer Sensor bekannt, der zur nicht-invasiven Messung von Blutparametern und insbesondere zur Messung des Blutflusses in einem Gefäßzugang dient. Die US 5,690,115 beschreibt eine Vorrichtung zur Messung des Blutflusses in einem Shunt mittels eines Doppler-Systems. Aus der JP 2002/315826 ist eine Bandage bekannt, die die Aufgabe hat, einen Shunt-Zugang aufgrund ihrer dämpfenden Eigenschaften zu schützen. Die DE 20 2012 005 321 U1 offenbart ein Diagnosepflaster mit einem Träger für elektronische Bauteile. Das Diagnosepflaster wird beispielsweise zur Erfassung von EKG-Daten verwendet. Die WO2010129528A2 betrifft ein Verfahren und System für die Überwachung der Sauerstoffzufuhr von Kompartimenten und Gewebe.

Der vorliegenden Erfindung liegt die Aufgabe zugrunde, eine Vorrichtung bereitzustellen, mittels derer zuverlässig Aussagen über den Funktionszustand eines Shunts getroffen werden können.

Diese Aufgabe wird durch eine Vorrichtung mit den Merkmalen des Anspruchs 1 gelöst.

Gemäß Anspruch 1 umfasst die Vorrichtung wenigstens eine durch den Patienten tragbare Bandage sowie eine Mehrzahl von in oder an der Bandage angeordneten Sensoren, die in zumindest einer zweidimensionalen Matrix angeordnet sind und die derart ausgebildet sind, dass sie eine mehrdimensionale Matrix von Messwerten wenigstens eines von den Sensoren erfassten Parameters erstellen. Die Sensoren können in die Bandage integriert sein oder sich an der Bandage, insbesondere an deren Oberfläche befinden.

Der Begriff "andage" ist allgemein zu verstehen und umfasst jede vorzugsweise am Arm des Patienten tragbare Manschette oder dergleichen, die geeignet ist, eine Mehrzahl von Sensoren aufzunehmen.

Jeder Shunt hat durch den pulsierenden Durchfluss ein typisches Bewegungs- und Dehnungsmuster. Diese pulsierende zeitliche Volumenänderung lässt sich mittels einer zweidimensionalen Sensormatrix, d.h. mit einer zweidimensionalen Anordnung von Sensoren gemäß der Erfindung gut erfassen.

Gemäß Anspruch 1, werden Dehnungssensoren verwendet , die in einer zweidimensionalen Matrix zur Erstellung eines raum-zeitlichen Musters der räumlichen, zwei- oder dreidimensionalen Dehnung der Hautoberfläche über dem Shunt angeordnet sind bzw. werden. Die mit dem Blutfluss oszillierende Gefäßausdehnung unmittelbar an dem Shunt lässt sich über beispielsweise in die Bandage eingearbeitete Dehnungssensoren erfassen.

Auch Piezosensoren in einer zweidimensionalen Matrix zur Erstellung eines raum-zeitlichen Musters der Dehnungskräfte der Hautoberfläche über dem Shunt sind denkbar und von der Erfindung mit umfasst.

Da die pulsierende und somit zeitliche veränderliche Volumenänderung mit Temperaturänderungen an der Hautoberfläche einhergeht, sind auch Temperatursensoren einsetzbar. Diese können in einer zweidimensionalen Matrix zur Erstellung eines raum-zeitlichen Musters der Temperatur an der Hautoberfläche über dem Shunt verwendet werden.

Ersatzweise sind auch optische Sensoren und insbesondere optische Reflexionssensoren einsetzbar, die in einer zweidimensionalen Matrix angeordnet sind. Diese Sensoren messen durch die Hautgewebeschichten und erfassen pulsierende Flussänderungen. Sie dienen zur Erstellung eines raum-zeitlichen Musters der Blutmenge in dem Bereich unterhalb der Hautoberfläche und vorzugsweise über dem Shunt. Als Lichtquelle dienen beispielsweise LEDs oder sonstige Licht im sichtbaren oder auch im IR-Bereich zur Messung der Blutabsorption abgebende Quellen. Die LEDs können beispielsweise in das Gewebe der Bandage eingebracht sein.

Vorzugsweise ist das Gewebe der Bandage für Licht bzw. für IR-Licht durchlässig. Unter einem raum-zeitlichen Muster ist ein zwei- oder mehrdimensionales Muster zu verstehen, das die Messwerte der Sensoren zeitgleich zu bestimmten Zeitpunkten oder permanent wiedergibt.

Über die ortsaufgelöste Amplitude oder das Über- oder Unterschreiten von Grenzwerten lässt sich ein Shunt in seiner korrekten oder mangelhaften Funktion charakterisieren und über die Änderung der gemessenen Werte lassen sich vorzugsweise Änderungen im örtlichen und/oder zeitlichen Flussverhalten des Blutes durch den Shunt erkennen.

Des Weitern sind erfindungsgemäß Bewegungs- und/oder Beschleunigungssensoren vorgesehen , die zur Artefaktunterdrückung von Bewegungen und Messsignalen aufgrund von Muskelanspannung beitragen und damit die Wahrscheinlichkeit für das Auftreten von Fehlmessungen verringern.

Weiterhin ist erfindungsgemäß ein oder mehrere die Herzaktivität messende Sensoren vorgesehen , nämlich als ein oder mehrere EKG Sensoren.

Da sich das Bewegungs- und Dehnungsmuster des Shunts bzw. der Haut in einer zeitlichen Korrelation mit dem EKG bzw. mit der Herztätigkeit befindet, wird über die Messung der Herztätigkeit mittels eines EKG-Sensors eine Zuordnung der Messwerte zu der Herztätigkeit vorgenommen werden. So dient die R-Zacke als Orientierungspunkt für die Messwerte bzw. für deren Vergleich mit Referenzwerten .

Die Sensoren können derart ausgebildet sein, dass diese wenigstens eine Eigenschaft der Haut erfassen. Alternativ oder zusätzlich können Sensoren verwendet werden, die wenigstens eine Eigenschaft eines unter der Haut liegenden Bereiches und insbesondere des Shunts erfassen.

Denkbar ist es weiterhin, dass die Vorrichtung derart ausgebildet ist, dass die Sensoren einen Absolutwert des gemessenen Parameters, wie beispielsweise die jeweils vorliegende Hauttemperatur erfassen. Von der Erfindung ist jedoch auch der Fall erfasst, dass die Sensoren einen Wert ausgeben, der für die Abweichung des gemessenen Wertes von einem oder mehreren Grenzwerten repräsentativ ist. So kann beispielsweise nur der Wert 0 oder 1 bzw. -1 und 1 bzw. die Farben schwarz oder weiß oder sonstige Farbkombinationen ausgegeben werden, je nachdem ob der gemessene Wert unter bzw. über einem Grenzwert liegt.

Die Vorrichtung kann eine Anzeigeeinrichtung, wie beispielsweise ein Display aufweisen, mittels derer die seitens der Sensoren gemessenen Werte bzw. darauf basierende Werte, wie z.B. Farben dargestellt werden. Diese Anzeigeeinrichtung kann mehrdimensional und insbesondere zweidimensional ausgebildet sein und die von den Sensoren jeweils gemessenen Werte nebeneinander, vorzugsweise in einer zweidimensionalen Matrix zu bestimmten Messzeitpunkten darstellen.

In der Ausgestaltung der Erfindung ist vorgesehen, dass die Vorrichtung zumindest eine Auswerteeinheit aufweist, wobei die Auswerteeinheit derart ausgebildet ist, dass sie die von den Sensoren erfassten Werte auswertet. Diese Auswertung liegt in einem Vergleich zwischen den gemessenen Werten und Sollwerten oder Sollwertbereichen .

Weiterhin ist vorgesehen, dass die Vorrichtung zumindest einen Speicher aufweist und dass in dem Speicher Referenzdaten für die mittels der Sensoren gemessenen Werte abgelegt sind.

Dabei ist die Auswerteeinheit derart ausgebildet , dass sie die von den Sensoren erfassten Werte mit den Referenzdaten vergleicht. Die Referenzdaten liegen als zwei- oder mehrdimensionales Muster vor. Auf der Grundlage dieses Vergleiches wird ein Signal oder eine Information z.B. an ein Dialysezentrum ausgegeben .

Denkbar ist es, dass ein typisches Funktionsbild, d.h. typische Sensorwerte gespeichert werden, und dieses dann in einem Speicher hinterlegt wird. Dieses Funktionsbild kann dann mit den Istwerten der Messungen verglichen und darauf basieren kann ein Rückschluss über den Funktionszustand des Shunts getroffen werden.

Dieses Funktionsbild kann in Abhängigkeit eines oder mehrerer Parameter, wie beispielsweise der Bewegung, der Herzrate, der Tageszeit etc. gespeichert werden und als Referenz für die folgende Überwachung herangezogen werden.

Verändert sich einer oder mehrere der gemessenen Werte dauerhaft und stetig von dem gespeicherten Referenzmodell, werden die erkannten Abweichungen vollautomatisch oder nach Aufforderung an den Patienten durch ihn ausgelesen und beispielsweise an ein Dialysezentrum übermittelt. In dem Dialysezentrum kann der behandelnde Arzt weitere Schritte, wie beispielsweise einen Untersuchungstermin zur bildgebenden Shuntkontrolle einleiten etc.

Erfindungsgemäß ist vorgesehen, dass die Vorrichtung eine Sendeeinheit aufweist, die mit der Auswerteeinheit in Verbindung steht und die derart ausgebildet ist, dass diese das Ergebnis der in der Auswerteeinheit vorgenommenen Auswertung an einen Empfänger drahtlos überträgt. Auf diese Weise ist eine für den Patienten kaum merkbare, dauerhafte oder zu bestimmten Zeitpunkten erfolgende Überwachung des Shunts in seinen Flusseigenschaften in der dialysefreien Zeit denkbar.

Die Sendeeinheit kann den Patienten und/oder ein Behandlungszentrum bzw. eine Klinik benachrichtigen, so dass der Shunt des Patienten schon vor der nächsten Behandlung im Dialysezentrum überprüft und ggf. korrigiert werden kann. Auf diese Weise lassen sich kritische Situationen bzw. eine Unterdialyse am Dialysetag vermeiden oder aufgrund der bekannten Situation des Shunts verringern. Abgesehen davon ist eine Anpassung der Dialyseparameter an den Funktionszustand des Shunts denkbar, so dass trotz einer Funktionsbeeinträchtigung eine ausreichende Dialysebehandlung durchgeführt werden kann.

Vorzugsweise ist vorgesehen, dass die Auswerteeinheit und/oder der Speicher in oder an der Bandage angeordnet ist. Denkbar ist somit eine "intelligente" Shuntbandage, die vorzugsweise noch die Funktion des Schutzes des Shunts vor mechanischen Einflüssen an dialysefreien Tagen hat.

Die Auswerteeinheit kann durch einen oder mehrere Mikrocontroller gebildet werden, die direkt oder per Kabel oder drahtlos an die Sensoren gekoppelt sind.

Die Auswerteeinheit und/oder der Speicher und/oder eine Energieversorgung können in die Bandage eingearbeitet oder an der Bandage angeordnet sein - entsprechend der Technologie der "wearable electronics".

Die Vorrichtung kann eine Energiequelle aufweisen, wobei vorzugsweise vorgesehen ist, dass die Energiequelle Peltier-Elemente aufweist oder aus diesen besteht. So ist beispielsweise die Stromversorgung der Auswerteeinheit über thermisches "Energy-Harvesting" über ein oder mehrere in die Bandage eingesetzte Peltier-Elemente möglich.

Die vorliegende Offenbarung betrifft des Weiteren ein Verfahren zur nicht invasiven Messung des Blutflusses durch einen Shunt eines Patienten, wobei das Verfahren mittels der Vorrichtung gemäß einem der Ansprüche 1 bis 5 durchgeführt wird und den Schritt des Anlegens der Bandage an den Patienten sowie die Erfassung von Messwerten wenigstens eines Parameters mittels der in einer mehrdimensionalen Matrix angeordneten Sensoren der Bandage umfasst.

Vorzugsweise umfasst das Verfahren den Schritt des Vergleiches der mittels der Sensoren erfassten Messwerte mit wenigstens einem Referenzwert, vorzugsweise mit wenigstens einem Referenzmuster. Dieses Referenzmuster kann als "Funktionsbild" des Shunts in Abhängigkeit eines oder mehrerer Parameter, wie beispielsweise der Bewegung, der Herzrate, der Tageszeit gespeichert und dann als Vergleichsmuster für die folgenden Überwachungen verwendet werden.

Denkbar ist es weiterhin, dass das Verfahren den Schritt der Übermittlung der Messwerte und/oder den Schritt der Übermittlung des Ergebnisses des Vergleichs zwischen den Messwerten und dem oder den Referenzmustern oder sonstigen Referenzwerten umfasst.

Weitere Vorteile und Einzelheiten der Erfindung werden anhand eines in der Zeichnung dargestellten Ausführungsbeispiels näher erläutert. Es zeigen:
- Figur 1:: eine exemplarische Darstellung eines zweidimensionalen Musters der mittels der Sensoren erfassten Drücke und
- Figur 2:: eine perspektivische Darstellung der Bandage gemäß der Erfindung.

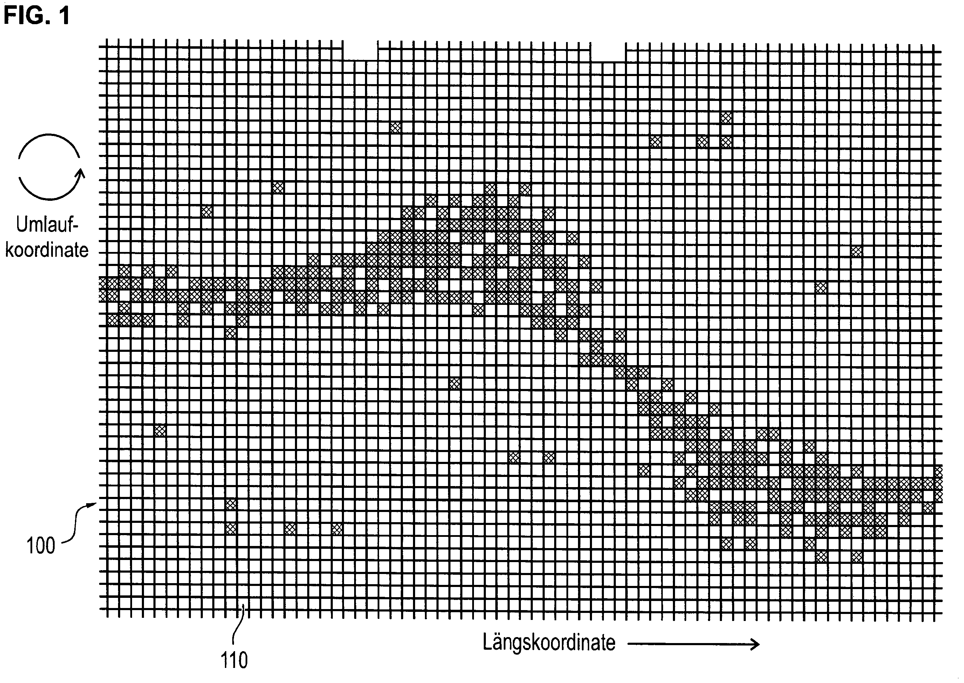

Das zweidimensionale Druckbild 100 gemäß Figur 1 wurde mittels einer zweidimensionalen Matrix von Sensoren ermittelt, deren Anzahl und Anordnung der der einzelnen Felder in Figur 1 entspricht.

Jeder einzelne Sensor erfasst den Druck auf der Haut des Patienten. Das Druckbild umfasst weiße und schwarze Kästchen 110, wobei jedes weiße Kästchen einem Druckwert entspricht, der unter einem Grenzwert liegt, und jedes schwarze Kästchen einem Druckwert entspricht, der über einem Grenzwert liegt. Die Längskoordinate stellt die laterale Richtung der Sensormatrix in Armrichtung dar und die Ordinate, d.h. die Umlaufkoordinate die Ausdehnung entlang des Umfangs des von der Bandage umwickelten Armes dar.

Wie dies aus Figur 1 hervorgeht, können auf diese Weise zweidimensionale Muster 100 zeitgleich erfasster Druckwerte generiert werden, bei denen räumliche Bereiche mit großen Druckwerten von räumlichen Bereichen mit kleinen Druckwerten differenziert werden können.

Denkbar ist weiterhin eine farbige Gestaltung der Felder, die beispielsweise Aufschluss über die Höhe des Druckes pro Sensorelement geben könnte.

Die Messung mit optischen, thermischen oder anderen Sensoren liefern ähnliche Funktionsbilder des Shunts.

Das in Figur 1 dargestellte Muster kann in einem Speicher hinterlegt werden und als Referenz für künftige Messungen verwendet werden. Weichen diese von dem Referenzmuster ab, kann dies als Indiz für eine Flussänderung durch den Shunt gewertet werden.

Das Muster kann zum Zwecke der Vergleichbarkeit stets zu einem bestimmten Zeitpunkt aufgenommen werden, beispielsweise mit oder unmittelbar nach dem Auftreten der R-Zacke des EKG.

Den Vergleich zwischen dem aufgenommenen Muster und einem Referenzmuster kann eine Auswerteeinheit vornehmen, die das Ergebnis der Auswertung zum Abruf bereitstellt oder an einem Empfänger, z.B. in einem Dialysezentrum sendet. Grundsätzlich kann die Auswertung direkt in der Bandage erfolgen oder auch entfernt von dieser. Im letzten Fall wird seitens einer Sendeeinheit nicht die Auswertung, sondern die Messwerte übermittelt.

In einer bevorzugten Ausgestaltung der Erfindung sind die Auswerteeinheit, z.B. in Form eines Microcontrollers, der Speicher und die Sensoren in die Bandage integriert oder an dieser angeordnet.

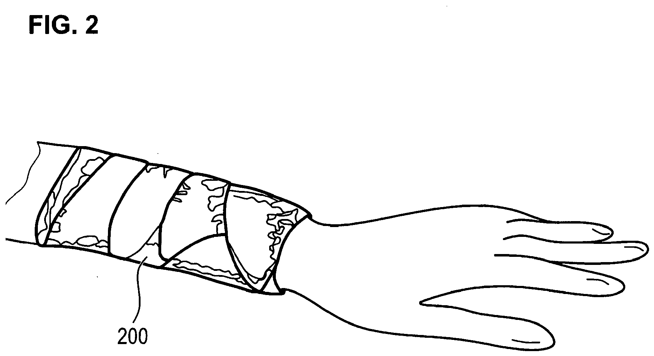

Eine solche Bandage ist in Figur 2 mit dem Bezugszeichen 200 dargestellt. In den dialysefreien Zeiten kann diese zum Schutz des Shunts vor äußeren Einflüssen dienen. Die Bandage 200 ist somit mehrfunktional, in dem sie einerseits einen mechanischen Schutz des Shunts bietet und andererseits eine mehrdimensionale Erfassung wenigstens eines Parameters vornimmt, die Rückschlüsse auf den Zustand des Shunts zulässt.

## Patentansprüche

1. Vorrichtung zur nicht-invasiven Messung des Blutflusses durch einen Shunt eines Patienten, umfassend
eine durch den Patienten tragbare Bandage (200), sowie eine Mehrzahl von in oder an der Bandage (200) angeordneten Sensoren, welche dazu ausgelegt sind, eine Dehnung der Haut zu erfassen,
ein oder mehrere in oder an der Bandage (200) angeordneten EKG Sensoren zur Erfassung eines Elektrokardiogramms,
einen Speicher, in dem Referenzdaten für die mittels der Sensoren erfassbaren Werte abgelegt sind, wobei die Referenzdaten als zwei- oder mehrdimensionales Muster vorliegen, und
eine Auswerteeinheit zum Auswerten der von den Sensoren erfassten Werte, wobei
die Sensoren in einer zweidimensionalen Matrix angeordnet sind und derart ausgebildet sind, dass sie ein raum-zeitliches Muster der räumlichen, zwei- oder dreidimensionalen Dehnung der Hautoberfläche über dem Shunt erstellen, wobei eine mit dem Blutfluss oszillierende Gefäßausdehnung unmittelbar an dem Shunt mittels der Sensoren stets zu oder unmittelbar nach dem Auftreten der R-Zacke des mittels des EKG Sensors gemessenen Elektrokardiogramms erfasst wird, und wobei das raum-zeitliche Muster ein zwei- oder mehrdimensionales Muster ist, das die Messwerte der Sensoren zeitgleich wiedergibt, und die Sensoren einen Wert ausgeben, der für die Abweichung des gemessenen Wertes von einem oder mehreren Grenzwerten repräsentativ ist, die Auswerteeinheit dazu ausgelegt ist, die von den Sensoren erfassten Werte mit den Referenzdaten zu vergleichen und auf Grundlage dieses Vergleichs ein Signal oder eine Information auszugeben,
wobei
eine Sendeeinheit, die mit der Auswerteeinheit in Verbindung steht und dazu ausgelegt ist, das Ergebnis der in der Auswerteeinheit vorgenommenen Auswertung an einen Empfänger drahtlos zu übertragen, wobei
die Vorrichtung ferner umfasst:
Bewegungs- und/oder Beschleunigungssensoren, die zur Artefaktunterdrückung von Bewegungen und Messsignalen aufgrund von Muskelanspannung beitragen.

2. Vorrichtung nach Anspruch 1, **dadurch gekennzeichnet, dass** die erstellte Matrix von Messwerten zweidimensional ist.

3. Vorrichtung nach einem der vorhergehenden Ansprüche, **dadurch gekennzeichnet, dass** die Sendeeinheit mit den Sensoren in Verbindung steht und derart ausgebildet ist, dass diese die von den Sensoren ermittelten Messwerte an einen Empfänger vorzugsweise drahtlos überträgt.

4. Vorrichtung nach einem der vorhergehenden Ansprüche, **dadurch gekennzeichnet, dass** die Auswerteeinheit und/oder der Speicher in oder an der Bandage angeordnet ist.

5. Vorrichtung nach einem der vorhergehenden Ansprüche, **dadurch gekennzeichnet, dass** die Vorrichtung eine Energiequelle aufweist, wobei vorzugsweise vorgesehen ist, dass die Energiequelle Peltier-Elemente aufweist oder aus diesen besteht.

## Claims

1. Apparatus for the non-invasive measurement of the blood flow through a shunt of a patient, comprising:
a bandage (200) which can be worn by the patient, as well as
a plurality of sensors which are arranged in or at the bandage (200), which are configured to detect a stretching of the skin,
one or more ECG sensors arranged in or on the bandage (200) for detecting an electrocardiogram,
a memory, in which reference data for the values detectable by means of the sensors stored are stored, wherein the reference data is present as a two or multidimensional pattern, and
an evaluation unit for evaluating the values detected by the sensors,
wherein
the sensors are arranged in a two-dimensional matrix and are configured such that they establish a space/time pattern of the spatial, two-dimensional or three-dimensional stretching of the skin surface on top the shunt, wherein a vascular dilatation oscillating with the blood flow is always detected together with or after occurrence of the R wave of the electrocardiogram measured by means of the ECG sensor, and wherein the space/time pattern is a two-dimensional or three-dimensional pattern that simultaneously reflects the measured values of the sensors, and the sensors output a value, which is representative of the deviation of the measured value to one or multiple threshold values, the evaluation unit is configured to compare the values detected by the sensors to the reference data and bases on this comparison, to output a signal or information,
**wherein**
a transmission unit which is in communication with the evaluation unit and is configured such that it transmits the result of the evaluation carried out in the evaluation unit wirelessly to a receiver, wherein
the apparatus further comprises:
movement and/or acceleration sensors, which contribute to suppressing artifacts of movements and measured signals due to muscle flexing.

2. Apparatus according to claim 1, **characterized in that** created matrix of measured values is two-dimensional.

3. Apparatus according to any one of the preceding claims, **characterized in that** the transmission unit is in communication with the sensors and is configured such that it transmits the measured values determined by the sensors to a receiver, preferably wirelessly.

4. Apparatus according to any one of the preceding claims, **characterized in that** the evaluation unit and/or the memory is arranged in or at the bandage.

5. Apparatus according to any one of the preceding claims, **characterized in that** the apparatus has an energy source, with provision preferably being made that the energy source comprises or consists of Peltier elements.

## Revendications

1. Dispositif pour la mesure non invasive du flux sanguin à travers un shunt d'un patient, comprenant
un bandage (200) pouvant être porté par le patient, et
une pluralité de capteurs agencés dans ou sur le bandage (200), qui sont conçus pour détecter une dilatation de la peau,
un ou plusieurs capteurs ECG agencés dans ou sur le bandage (200) pour détecter un électrocardiogramme,
une mémoire dans laquelle sont stockées des données de référence pour les valeurs pouvant être détectées au moyen des capteurs, les données de référence étant présentes sous la forme d'un modèle bidimensionnel ou multidimensionnel, et
une unité d'évaluation pour évaluer les valeurs détectées par les capteurs,
les capteurs étant agencés dans une matrice bidimensionnelle et étant configurés de telle sorte qu'ils établissent un modèle spatio-temporel de la dilatation spatiale, bidimensionnelle ou tridimensionnelle de la surface de la peau au-dessus du shunt, une dilatation vasculaire oscillant avec le flux sanguin étant détectée directement au niveau du shunt au moyen des capteurs toujours à l'apparition ou directement après l'apparition de l'onde R de l'électrocardiogramme mesuré au moyen du capteur ECG, et le modèle spatio-temporel étant un modèle bidimensionnel ou multidimensionnel qui reproduit simultanément les valeurs de mesure des capteurs, et les capteurs émettant une valeur qui est représentative de l'écart de la valeur mesurée par rapport à une ou plusieurs valeurs limites, l'unité d'évaluation étant conçue pour comparer les valeurs détectées par les capteurs aux données de référence et pour émettre un signal ou une information sur la base de cette comparaison,
**dans lequel**
une unité d'émission, qui est en communication avec l'unité d'évaluation et qui est conçue pour transmettre sans fil le résultat de l'évaluation effectuée dans l'unité d'évaluation à un récepteur,
le dispositif comprenant en outre :
des capteurs de mouvement et/ou d'accélération qui contribuent à la suppression d'artefacts de mouvements et de signaux de mesure dus à la tension musculaire.

2. Dispositif selon la revendication 1, **caractérisé en ce que** la matrice de valeurs de mesure établie est bidimensionnelle.

3. Dispositif selon l'une quelconque des revendications précédentes, **caractérisé en ce que** l'unité d'émission est en communication avec les capteurs et est configurée de telle sorte qu'elle transmet les valeurs de mesure déterminées par les capteurs à un récepteur, de préférence sans fil.

4. Dispositif selon l'une quelconque des revendications précédentes, **caractérisé en ce que** l'unité d'évaluation et/ou la mémoire sont agencées dans ou sur le bandage.

5. Dispositif selon l'une quelconque des revendications précédentes, **caractérisé en ce que** le dispositif présente une source d'énergie, il étant de préférence prévu que la source d'énergie présente des éléments Peltier ou est constituée de ceux-ci.
